# EUROPÄISCHE PATENTSCHRIFT

(11) **EP 2 679 229 B1**
(45) Veröffentlichungstag und Bekanntmachung des Hinweises auf die Patenterteilung: **27.02.2019**
(21) Anmeldenummer: 12174534.3
(22) Anmeldetag: 30.06.2012
(51) Int. Cl.: A61K 31/7034, A61K 36/31, A61P 3/10

(54) **Zusammensetzung zur Behandlung von hyperglykämischen Erkrankungen**
Composition for the treatment of hyperglycemic diseases
Composition pour le traitement de pathologies hyperglycémiques

(43) Veröffentlichungstag der Anmeldung: 01.01.2014
(73) Patentinhaber: BioActive Food GmbH, 23795 Bad Segeberg (DE)
(72) Erfinder: Vollert, Henning, 23795 Bad Segeberg (DE)
(74) Vertreter: Uexküll & Stolberg

(56) Entgegenhaltungen:
- JP-A- 2008 074 745
- DATABASE WPI Week 200830 Thomson Scientific, London, GB; AN 2008-E39694 XP002681442, & JP 2008 074742 A (KAO CORP) 3. April 2008 (2008-04-03)
- DATABASE WPI Week 200377 Thomson Scientific, London, GB; AN 2003-820627 XP002681443, & JP 2003 238417 A (EBATA T) 27. August 2003 (2003-08-27)

## Beschreibung

Die vorliegende Erfindung betrifft eine synergistisch wirkende Zusammensetzung, die (a) mindestens einen Extrakt einer Pflanze der Gattung *Brassica* und (b) mehr als 1% (w/w) Phlorizin enthält, wobei es sich bei der Pflanze der Gattung *Brassica* um Grünkohl handelt. Insbesondere betrifft die vorliegende Erfindung eine solche Zusammensetzung zur Verwendung in einem Verfahren zur Behandlung und/oder Prophylaxe einer hyperglykämischen Erkrankung, wie z.B. Fettsucht, Diabetes oder einer von Diabetes verursachten Folgeerkrankung. Die Erfindung betrifft ferner die Verwendung einer solchen Zusammensetzung zur Herstellung einer pharmazeutischen Zusammensetzung, eines diätetischen Lebensmittels und/oder eines Nahrungsergänzungsmittels. Die Erfindung betrifft darüber hinaus pharmazeutische Zusammensetzungen, diätetische Lebensmittel und Nahrungsergänzungsmittel, die eine Zusammensetzung gemäß der vorliegenden Erfindung umfassen.

### GEBIET DER ERFINDUNG

Die Zahl der Diabetes-Toten ist nach Angaben des Statistischen Bundesamts seit 1980 um 29 Prozent gestiegen. Diabetes mellitus ist damit verantwortlich für knapp drei Prozent aller Sterbefälle (1980: zwei Prozent) in Deutschland. Parallel dazu steigt die Zahl der übergewichtigen Menschen in Deutschland stark an; jeder zweite Deutsche ist bereits übergewichtig, jeder fünfte sogar fettleibig. Bei diesen Menschen ist das Risiko, an Diabetes zu erkranken, dramatisch erhöht.

Aktuelle Hochrechnungen gehen davon aus, dass bis zu 7 % der Deutschen (Hauner H. Dtsch Med Wochenschr 2005; 130 Suppl 2:S.64-65), d.h. rund 6 Millionen Menschen, wegen eines Diabetes in Behandlung sind. Die Anzahl der Neuerkrankungen nimmt dabei mit steigendem Alter zu; in der Altersgruppe der über 60-jährigen liegt der Anteil der an Diabetes Erkrankten bei 18-28 %. Parallel dazu sinkt das Manifestationsalter betroffener Typ-2-Diabetiker stetig. Typ-2-Diabetes ist mit rund 11 % die vierthäufigste Diagnose der hausärztlichen Internisten und mit rund 8 % die fünfthäufigste Diagnose aller Allgemeinärzte (Wittchen HU, (HYDRA)-Studie, Fortschr Med Orig 2003; 121). Aktuelle Schätzungen der WHO gehen davon aus, dass sich die Zahl der an Diabetes Erkrankten bis zum Jahr 2025 verdoppeln wird (World Health Organization, Diabetes mellitus, Fact sheet No. 138, 2002).

Mit der Nahrung wird eine Vielzahl an Fetten, Kohlehydraten und Proteinen aufgenommen, die den menschlichen Körper mit der benötigten Energie versorgen. Eine zu starke Aufnahme und insbesondere eine zu schnelle Aufnahme von Zuckern stellt eine starke Belastung des Stoffwechsels dar und gilt als ein wesentlicher Risikofaktor für Diabetes, Übergewicht und Fettleibigkeit. Es besteht daher ein anhaltender Bedarf an neuen Mitteln für die Behandlung von hyperglykämischen Erkrankungen wie Diabetes und Fettleibigkeit.

Zahlreiche Verbindungsklassen sind für die Behandlung des Diabetes mellitus, insbesondere des Diabetes mellitus Typ II, vorgeschlagen worden. In verschiedenen Internationalen Anmeldungen sind beispielsweise Substanzen vorgeschlagen worden, die den Natrium-abhängigen Glucose-Transporter 2 (SGLT-2) hemmen (siehe z.B. WO 98/31697, WO 02/083066, WO 03/099836 und WO 01/31697). Dieses Transportprotein resorbiert im proximalen Tubulus der Niere Glucose und Natrium aus dem Primärharn. Spezifische SGLT-2-Inhibitoren sind in der Lage, die Aufnahme von Glucose in den Nierentubuli zu verhindern, was zur Wiederherstellung von normalen Plasmaglucosespiegeln führt. Eine langfristige Behandlung mit SGLT-2-Inhibitoren von mehr als 6 Monaten hat im Tierversuch zu einer verstärkten Insulin-Empfindlichkeit, einer verbesserten Insulin-Reaktion und zu einer verzögerten Ausbildung von Diabetes-bedingten Komplikationen geführt. Die Inhibierung des Transporters SGLT-2 beruht auf einer verstärkten konzentrationsabhängigen Ausscheidung von Glucose, was zu einer Senkung des Blutzuckers im Körper führt. Dabei wird jedoch keine Hypoglykämie verursacht. Der Mechanismus der Blutzuckersenkung ist unabhängig von einer ggf. bestehenden Insulinresistenz oder einer mangelnden Insulinproduktion durch die Betazellen der Bauchspeicheldrüse. Neben SGLT-2 gibt es einen weiteren Natrium-abhängigen Glucose-Transporter, SGLT-1, der ebenfalls mit der Behandlung des Diabetes in Verbindung gebracht wird. SGLT-1 wird im Dünndarm sowie im S3-Segment des proximalen Nierentubulus gefunden. Er ist für die aktive Aufnahme von Glucose verantwortlich. Im Vergleich zu SGLT-2 weist SGLT-1 für einige Zucker eine veränderte Substratspezifität auf. Verschiedene Präparate, die zu einer Hemmung von SGLT-1 und/oder SGLT-2 führen, befinden sich derzeit in klinischen Erprobungen.

Im Rahmen der vorliegenden Erfindung wurde festgestellt, dass die orale Gabe eines Extrakts aus einer Pflanze der Gattung *Brassica* oder eines Extrakts aus Früchten der Gattung *Malus* (Äpfel) jeweils zu einer leichten Hemmung des Natrium-abhängigen Glucose-Transporters SGLT-1 führt. Überaschenderweise führte die gleichzeitige Gabe dieser Extrakte zu einer deutlich stärkeren Inhibierung des SGLT-1 und damit zur verringerten Aufnahme von Zucker aus im Darm. Die synergistische Wirkung der beiden Extrakte ist auf das Zusammenwirken des Brassica-Extrakts mit dem im Apfelextrakt enthaltenen Phlorizin zurückzuführen. Demgemäß eignet sich eine Zusammensetzung, die (a) einen Extrakt aus einer Pflanze der Gattung *Brassica* und (b) Phlorizin enthält, in hohem Maße für die Behandlung und/oder Prophylaxe einer hyperglykämischen Stoffwechselerkrankung wie Diabetes und/oder Übergewicht. Eine solche Zusammensetzung führt über die Hemmung des Natrium-abhängigen Glucose-Transporters zu deutlich geringeren Erhöhungen der Blutglucosekonzentration nach der Aufnahme von Lebensmitteln, die Kohlenhydrate, wie z.B. Stärke, enthalten.

JP 2008 074742 A und JP 2008 074745 A beschreiben die Verwendung von isoliertem Brassinin in Kombination mit Phloretin oder Phloretin-Glykosiden zur Verringerung des Blutglucose-Spiegels. JP 2003 238417 A beschreibt die Verwendung von Phlorizin und beta-Glucosidase-Inhibitoren als therapeutisches Mittel gegen Diabetes.

### BESCHREIBUNG DER ERFINDUNG

Demgemäß betrifft die Erfindung in einem ersten Aspekt Zusammensetzungen, die mindestens die folgenden beiden Bestandteile enthalten: (a) mindestens einen Extrakt aus einer Pflanze der Gattung *Brassica,* wobei es sich bei der Pflanze der Gattung *Brassica* um Grünkohl handelt und (b) mehr als 1% (w/w) Phlorizin. Die erfindungsgemäßen Zusammensetzungen werden erfindungsgemäß für die Behandlung und/oder die Prophylaxe einer hyperglykämischen Erkrankung vorgeschlagen. Die Zusammensetzungen zeichnen sich durch eine synergistische Wirkung ihrer Bestandteile aus, d.h. das Zusammenwirken der einzelnen Bestandteile geht über einen rein additiven Effekt hinaus.

Erkrankungen, die sich mit Hilfe der erfindungsgemäß vorgeschlagenen Zusammensetzungen behandeln lassen, umfassen alle Krankheiten, die durch eine Hyperglykämie, d.h. durch einen erhöhten Blutzuckerspiegel gekennzeichnet sind, der den physiologisch normalen Wert von 140 mg/dl (7,8 mmol/l) übersteigt. Solche Erkrankungen umfassen insbesondere Fettsucht, Diabetes oder von Diabetes verursachte Folgeerkrankungen (wie z.B. Retinopathie, Neurophathie, Nephropathie und gestörte Wundheilung). Bei dem Diabetes, der mittels der erfindungsgemäßen Zusammensetzungen behandelt werden soll, kann es sich um Diabetes Typ I oder Typ II handeln. Vorzugsweise handelt es sich um Diabetes Typ II. Ferner werden die erfindungsgemäßen Zusammensetzungen allgemein für die Behandlung und/oder Prophylaxe solcher Erkrankungen und Zustände vorgeschlagen, bei denen es vorteilhaft ist, die Zuckeraufnahme aus dem Darm zu verlangsamen und/oder zu reduzieren.

Die erfindungsgemäß zur Behandlung oder Prophylaxe vorgeschlagenen Zusammensetzungen können auch in vorteilhafter Weise mit anderen Wirkstoffen verwendet werden, z.B. zusammen mit anderen im Stand der Technik beschriebenen antidiabetischen Mitteln, wie z.B. Biguaniden, Sulfonylharnstoffen, Glucosidaseinhibitoren, Thiazolidindionen, Dipeptidylpeptidase IV (DP4)-Inhibitoren, Meglitiniden, Glucagon-ähnliches Peptid-1 (GLP-1), PTP1B-Inhibitoren, Glycogenphosphorylase-Inhibitoren und Glucose-6-Phosphatase-Inhibitoren.

Bei der Gattung *Brassica* handelt es sich um eine pflanzliche Gattung innerhalb der Familie der Kreuzblütengewächse (Brassicaceae). Besonders bevorzugt ist es, dass der oder die *Brassica*-Extrakt(e) ausgehend von Pflanzen der Spezies *Brassica oleracea* hergestellt wird. Bei der Spezies *Brassica oleracea* handelt es sich um eine formenreiche Pflanzenart innerhalb der Gattung *Brassica.* Die Art umfasst zahlreiche Sorten, zu denen u.a. Brokkoli *(Brassica oleracea* var. *silvestris* L.), Rotkohl *(Brassica oleracea* var. *capitata f. rubra* L.), Weißkohl *(Brassica oleracea* var. *capitata f. alba),* Wirsing *(Brassica oleracea L. convar. capitata (L.) Alef. var. sabauda L.*), Chinakohl (*Brassica rapa* ssp. *pekinensis*) und Grünkohl (*Brassica oleracea* convar. *acephala* var. *sabellica*) zählen. Erfindungsgemäß wird der oder die Extrakt(e) aus einer Pflanze der Gattung *Brassica* ausgehend von Grünkohl hergestellt Die erfindungsgemäßen Zusammensetzungen umfassen neben einem oder mehreren Brassica-Extrakten das Flavonoid Phlorizin in einer Menge von mehr als 1% (w/w). Das Phlorizin stammt dabei nicht aus dem Brassica-Extrakt, sondern wird diesem zugesetzt, z.B. in Form eines Phlorizin-haltigen Pflanzenextrakts. Grünkohl enthält kein oder nur sehr wenig (>0,0001% (w/w)) Phlorizin. Phlorizin hemmt den SGLT *in vitro* (Kottra et al. (2007), J Pharmacol Exp Ther, 322 (2) :829-35). Es ist ferner seit langer Zeit bekannt, dass Phlorizin auf die Glucoseausscheidung der Niere wirkt, wenn es intravenös appliziert wird. Allerdings ist die Wirkung schwach, wenn Phlorizin oral eingenommen wird. Dies ist vermutlich auf einen enzymatischen Abbau des Phlorizins im Darm zurückzuführen, der z.B. durch das Enzym Laktat-Phlorizin-Hydrolase katalysiert wird (Birkenmeier & Alpers (1974), Biochim Biophys Acta., 350(1):100-12). Offenbar wird nach Einnahme der erfindungsgemäßen Zusammensetzung der enzymatische Abbau des Phlorizins im Darm durch bestimmte Bestandteile des Brassica-Extrakts gehemmt, wodurch das Phlorizin seine inhibitorischen Eigenschaften auf die Glucoseaufnahme (SGLT 1) entfalten.

Die erfindungsgemäßen Zusammensetzungen enthalten eine Menge an Phlorizin, die wirksam ist, um nach oraler Gabe den Glucose-Transporters SGLT-1 zu hemmen und/oder die Zuckeraufnahme aus dem Darm zu verlangsamen. In einer bevorzugten Ausführungsform wird die Aktivität des im Darm befindlichen SGLT-1-Transporters durch die Zusammensetzungen um mindestens etwa 1%, 2%, 3%, 4%, 5%, 10%, 20%, 30%, 40%, 50%, 60%, 70%, 80%, 90% oder mehr gegenüber dem unbehandelten Zustand verringert. In einer weiteren bevorzugten Ausführungsform führt die erfindungsgemäße Zusammensetzung nach Gabe zu einer Zuckeraufnahme aus dem Darm die gegenüber dem unbehandelten Zustand um mindestens etwa 1%, 2%, 3%, 4%, 5%, 10%, 20%, 30%, 40%, 50%, 60%, 70%, 80%, 90% oder mehr verringert ist.

Der Fachmann ist problemlos in der Lage, die für die jeweils zu erreichende (therapeutische) Wirkung erforderliche Menge an Phlorizin durch einfache Versuchsreihen zu ermitteln. Hierbei wird der Fachmann verschiedene Faktoren berücksichtigen, wie z.B. die Art und Menge der in der Zusammensetzung vorhandenen Brassica-Extrakts, die Art und Menge der zusätzlich in den Zusammensetzungen vorhandenen Komponenten, Alter und Gewicht der Person, an die die Verabreichung der Zusammensetzung erfolgt, usw. Die Menge an Phlorizin in den erfindungsgemäßen Zusammensetzungen ist größer als 10⁴ ppm, vorzugsweise größer als 5*10⁴ ppm oder größer als 10⁵ ppm. Anders ausgedrückt enthält die erfindungsgemäße Zusammensetzung mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, mehr als 14%, oder mehr als 15%. In einer besonders bevorzugten Ausführungsform enthalten die erfindungsgemäßen Zusammensetzungen mehr als 5% Phlorizin. Wie hierin verwendet, bezeichnet die Angabe "%" Gewichtsprozent (w/w).

Die erfindungsgemäße Zusammensetzung wird vorzugsweise so formuliert, dass die jeweilige Verabreichungseinheit (z.B. eine Kapsel oder Tablette) eine Menge von 0,01 und 10 g Phlorizin enthält, z.B. mehr als 0,05 g, mehr als 0,1 g, mehr als 0,2 g, mehr als 0,3 g, mehr als 0,4 g, mehr als 0,5 g, mehr als 0,75 g, mehr als 1 g, mehr als 2 g, mehr als 3 g, mehr als 4 g oder mehr als 5 g.

In einer besonders bevorzugten Ausführungsform betrifft die Erfindung eine Zusammensetzung, in der das Phlorizin in Form eines Phlorizin-haltigen pflanzlichen Extrakts vorliegt. Der Phlorizin-haltige pflanzliche Extrakt kann z.B. aus mindestens einer Pflanze der Familie *Rosaceae* gewonnen werden. Vorzugsweise gehört die Pflanze aus der Familie *Rosaceae* dabei einer Gattung an, die ausgewählt ist aus der Gruppe bestehend aus *Malus, Pyrus* oder *Prunus.* Der Phlorizin-haltige pflanzliche Extrakt kann ferner auch aus einer Pflanze der Familie *Verbenaceae* gewonnen werden. Vorzugsweise handelt es sich bei der Pflanze aus der Familie *Verbenaceae* um eine solche der Gattung *Lippia.* Es ist bekannt, dass insbesondere Pflanzen dieser Familien Phlorizin enthalten. Der Extrakt der Pflanze aus der Familie der *Rosaceae* oder *Verbenaceae* kann dabei z.B. aus der Rinde, den Früchten oder den Blättern der Pflanze gewonnen wurde.

Pflanzen der Gattungen *Malus, Pyrus* und *Prunus* enthalten relativ hohe Mengen an Phlorizin in der Rinde, in den Früchten und in den Blättern. Insbesondere die Früchte der Gattung *Malus* (d.h. Äpfel) weisen besonders hohe Mengen an Phlorizin auf. Es ist daher besonders bevorzugt, dass das Phlorizin in der erfindungsgemäßen Zusammensetzung in Form eines Extrakts aus einer Pflanze der Gattung *Malus,* vorzugsweise in Form eines Extrakts aus Äpfeln oder der Rinde, vorliegt. Bei der Gattung *Malus* handelt es sich um eine pflanzliche Gattung in der Familie der Rosengewächse (Rosaceae). Besonders bevorzugt ist es, dass die Extrakte aus einer Pflanze der Gattung *Malus* ausgehend von Pflanzen der Spezies *Malus domestica* hergestellt werden. Der Kulturapfel (*Malus domestica* Borkh.; *Pyrus malus,* L.) ist eine wirtschaftlich sehr bedeutende Obstart, zu der zahlreiche Sorten gehören. Grundsätzlich können alle Sorten des Kulturapfels zur Herstellung der Phlorizin-haltigen Extrakte verwendet werden. Besonders bevorzugt ist es, dass die Extrakte ausgehend von einer der Sorten Red Delicious, Golden Delicious, Braeburn, Cox Orange, Finkenwerder Prinz, Fürst Blücher, Märkischer Cox oder Red Chief hergestellt werden. Neben Pflanzen der Spezies *Malus domestica* können jedoch auch andere Spezies der Gattung *Malus* Verwendung finden.

Es ist bevorzugt, dass der mindestens eine Extrakt aus der Pflanze aus der Familie der *Rosaceae* und/oder der mindestens eine Extrakt aus der Familie der *Verbenaceae* vorzugsweise mindestens 2%, 3%, 4%, 5%, 6%, 7%, 8%, 9%, 10%, 15%, 20% oder mehr Phlorizin enthält (w/w).

In einer besonders bevorzugten Ausführungsform umfassen die Zusammensetzungen mindestens einen Extrakt aus einer Pflanze der Gattung *Brassica,* wobei es sich bei der Pflanze der Gattung *Brassica* um Grünkohl handelt, und mindestens einen Extrakt aus mindestens einer Pflanze der Familie *Rosaceae* und/oder aus mindestens einer Pflanze der Familie *Verbenaceae.* In einer Ausführungsform enthalten die Zusammensetzungen mehr als einen, z.B. zwei, drei, vier oder mehr, Extrakte aus einer Pflanze der Gattung *Brassica* und/oder mehr als einen, z.B. zwei, drei, vier oder mehr, Extrakte aus einer Pflanze der Gattung *Malus.* Besonders bevorzugt sind Zusammensetzungen, die einen Grünkohl-Extrakt und mindestens einen Extrakt aus einer Pflanze der Gattung *Malus* enthalten. Der hier verwendete Begriff "Extrakt aus einer Pflanze" bezeichnet einen Extrakt der aus einer oder mehreren Pflanzen der gleichen Art bzw. Spezies gewonnen wird und ist nicht als auf eine einzige Pflanze beschränkt zu verstehen.

Wie vorliegend verwendet bezeichnet der Begriff "Extrakt" ein Stoffgemisch, das durch Aufschluss von Zellen einer Pflanze und Gewinnung des Zellsafts erhalten wird. Die erfindungsgemäß vorgeschlagenen Extrakte lassen sich durch eine Vielzahl von im Stand der Technik bekannten Verfahren herstellen.

In einer Ausführungsform der Erfindung wird zunächst Material einer Pflanze bereitgestellt. Als Ausgangsmaterial für das erfindungsgemäße Verfahren eignen sich Pflanzen in sämtlichen Entwicklungsstadien, vom Samen bis zur reifen Pflanze. Ferner eignen sich sämtliche Pflanzenteile, wie z.B. die Früchte, Stängel, Blätter, Rinde oder Wurzeln der Pflanze. Das Ausgangsmaterial kann eine Mischung aus verschiedenen Pflanzenteilen sein, oder nur bestimmte Pflanzenteile umfassen. Das pflanzliche Material wird in der Regel zunächst zerkleinert. Das Zerkleinern kann durch einfaches Zerstückeln der jeweiligen Pflanzenteile mit herkömmlichen Cuttern erreicht werden. Es lassen sich grundsätzlich alle Pflanzenteile als Ausgangspunkt für die Extraktbildung verwenden. Bei der Herstellung von Brassica-Extrakten werden besonders gute Ergebnisse erzielt, wenn Blätter der Pflanzen verwendet werden. Daher werden die Extrakte aus einer Pflanze der Gattung *Brassica* gemäß einer bevorzugten Ausführungsform der Erfindung aus den Blättern der jeweiligen Brassica-Pflanze hergestellt. Bei der Herstellung von Extrakten von Pflanzen der Gattung *Malus* haben sich die Früchte und die Rinde als besonders geeignet erwiesen. In einer weiteren bevorzugten Ausführungsform werden die Extrakte aus einer Pflanze der Gattung *Malus* aus den Früchten, d.h. den Äpfeln, oder aus der Rinde der jeweiligen Malus-Pflanze hergestellt. Vor der Zerkleinerung kann das pflanzliche Material bei Temperaturen zwischen 45°C und 100°C blanchiert werden, um bakterielle Verunreinigungen zu beseitigen und die Qualität des Aufschlusses zu verbessern. Des Weiteren wird durch das Blanchieren die Aktivität von Enzymen wie Hydrolasen, Lipasen und Oxidasen reduziert und somit die Stabilität und Qualität des Pflanzenmaterials erhöht.

In einem nächsten Schritt wird das pflanzliche Material aufgeschlossen, d.h. die zellulären Strukturen des Materials werden zerstört, sodass der Inhalt der Zellen freigesetzt wird. Der Aufschluss kann durch herkömmliche Verfahren zum Aufschluss pflanzlicher Zellen erreicht werden, beispielsweise durch wiederholtes Einfrieren und Auftauen, oder durch geeignete Apparaturen, wie z.B. durch Homogenisatoren, Hochdruckhomogenisatoren oder Ultraschallhomogenisatoren. Auch Kolloidmühlen oder French-Pressen können für den Aufschluss verwendet werden.

Darüber hinaus kann der Zellaufschluss auch enzymatisch erzielt werden. Zu diesem Zweck wird das pflanzliche Material mit geeigneten Enzymen versetzt, die zu einer Zerstörung der strukturellen Bestandteile der Zellen führen. Es hat sich gezeigt, dass eine Inkubation des pflanzlichen Materials mit Pektinase, Kollagenase, Cellulase und/oder Hemicellulasen in dieser Phase des Verfahrens besonders geeignet ist, die Zellen wirkungsvoll aufzuschließen. Die Inkubationszeit kann zwischen 30 min und 24 Stunden liegen, vorzugsweise zwischen 1 Stunde und 6 Stunden, mehr bevorzugt zwischen 90 min und 4 Stunden, z.B. 2 Stunden. Die Temperatur kann im Bereich von 20° und 60°C liegen, vorzugsweise zwischen 25°C und 45°C, z.B. bei ca. 37°C, und sie sollte vorzugsweise im Bereich des Temperaturoptimums des jeweils verwendeten Enzyms liegen. Ein geeigneter pH-Wert des Reaktionsansatzes kann unter Verwendung geeigneter Puffer, wie z.B. Phosphat-, Carbonat-, Natriumhydrogencarbonatpuffer und/oder geeigneter Säuren, wie z.B. Citronensäure, Milchsäure oder Ascorbinsäure eingestellt werden. Ein pH Wert zwischen 4 und 6, z.B. pH 5, ist für den Aufschluss besonders geeignet.

Nach Aufschluss der Zellen, kann der erhaltende Zellsaft direkt zur Hemmung des Natrium-abhängigen Glucose-Transporters 1 (SGLT-1) verwendet werden. Es ist allerdings bevorzugt, den nach dem Aufschluss der Zellen erhaltenen Pflanzensaft weiteren Reinigungsschritten zu unterziehen.

Beispielsweise kann das aus dem Zellaufschluss erhaltene Material einer Trocknung oder Gefriertrocknung mit anschließender Extraktion unterzogen werden. Hier kann beispielsweise zunächst eine Sprühtrocknung eingesetzt werden. Das getrocknete oder gefriergetrocknete pflanzliche Material kann anschließend mit einem wässrigen oder organischen Lösungsmittel extrahiert werden. Die Extraktion kann mit einem herkömmlichen Extraktionsmittel durchgeführt werden, z.B. mit Ethanol, Ethylacetat oder mit anderen organischen Lösungsmitteln. Auch eine Extraktion mit Gasen, wie z.B. mit Stickstoff ist möglich. Bei Verwendung eines flüssigen Lösungsmittels beträgt die Inkubationszeit 0,5 bis 24 Stunden, vorzugsweise 2 bis 8 Stunden. Bei Verwendung von Stickstoff erfolgt die Extraktion bei Temperaturen zwischen 4°C und 37°C in einem Zeitraum von 0,25 bis 3 Stunden, vorzugsweise in einem Zeitraum von 20 und 60 Minuten.

Alternativ zu einer Trocknung/Extraktion kann der nach Aufschluss der Zellen erhaltene Pflanzensaft auch einer chromatographischen Reinigung unterzogen werden, um die Polyphenole (einschließlich der Flavonoide) anzureichern, beispielsweise unter Verwendung einer Ionenaustauschchromatographie oder einer Affinitätschromatographie. Wie in den Beispielen beschrieben wird, kann zu diesem Zweck z.B. eine XAD 16HD-Säule (Fa. Rohm & Haas, Frankfurt) verwendet werden. Die Säule wird mit dem aus dem Zellaufschluss erhaltenen Überstand beladen, und durch Spülen mit demineralisiertem Wasser werden hydrophile Substanzen abgereichert. Die Polyphenole dagegen können durch Elution mit Ethanol angereichert werden. Die eluierten Fraktionen können anschließend direkt verwendet oder getrocknet werden. So können die eluierten Fraktionen z.B. einer Gefriertrocknung unterzogen werden, und das dabei erhaltene Pulver kann für die Verabreichung verkapselt werden

Die erfindungsgemäß vorgeschlagenen Zusammensetzungen lassen sich durch eine Vielzahl von im Stand der Technik bekannten Verfahren herstellen. So können beispielsweise die von der Zusammensetzung umfassten Extrakte aus den Pflanzen der Gattungen *Brassica* und *Malus* zunächst einzeln hergestellt und unmittelbar vor der Verwendung zu einer die Extrakte umfassenden Zusammensetzung gemischt werden. Alternativ dazu können zunächst ein oder mehrere *Brassica*-Extrakte hergestellt und miteinander vermischt werden, wobei anschließend die Zugabe von isoliertem Phlorizin erfolgt. Phlorizin kann durch im Stand der Technik bekannte Verfahren hergestellt oder von verschiedenen Herstellern direkt erworben werden (z.B. von der Carl Roth GmbH & Co. KG, Karlsruhe, Deutschland).

In einer bevorzugten Ausführungsform wird das Phlorizin dem Grünkohl-Extrakt in Form eines weiteren pflanzlichen Extrakts zugegeben. In einer besonders bevorzugten Ausführungsform handelt es sich bei dem Phlorizin-haltigen Extrakt um einen Extrakt aus einer Pflanze der Gattung *Malus.*

Die erfindungsgemäßen Zusammensetzungen, die mindestens einen Grünkohl-Extrakt sowie Phlorizin enthalten, werden vorliegend zur Behandlung und/oder zur Prophylaxe einer hyperglykämischen Erkrankung vorgeschlagen. Dabei spielt es keine Rolle wann die einzelnen Komponenten miteinander gemischt werden, so lange sie zeitnah verabreicht werden. Daher betrifft die vorliegende Erfindung auch die Verwendung von mindestens einem Grünkohl-Extrakt und mindestens einem Phlorizin-enthaltenen Extrakt bzw. Phlorizin zur Behandlung und/oder zur Prophylaxe einer hyperglykämischen Erkrankung, wobei die Komponenten für die getrennte Verabreichung formuliert sind.

Sofern das Phlorizin dem Grünkohl-Extrakt in Form eines Phlorizin-enthaltenen Pflanzenextrakts zugefügt wird, können die beiden Extrakte in jeglichem Mischungsverhältnis verwendet werden. In einer Ausführungsform enthält die erfindungsgemäße Zusammensetzung den Grünkohl-Extrakt und den Phlorizin-enthaltenen Extrakt im Verhältnis von ungefähr 20:1, 10:1, 5:1, 4:1, 3:1, 2:1, 1:1, 1:2, 1:3, 1:4, 1:5, 1:10 oder 1:20. Ein Mischungsverhältnis zwischen dem Grünkohl-Extrakt und dem Phlorizin-enthaltenen Extrakt von 1:1 ist besonders bevorzugt.

Es wird angenommen, dass die den SGLT-1 hemmenden Bestandteile des Grünkohl-Extrakts zur Gruppe der Flavonoide und/oder Glycoside gehören. Flavonoide zählen zur Gruppe der sekundären Pflanzenstoffe, und sie stellen eine umfangreiche Stoffklasse polyphenolischer Verbindungen dar, die in Lebensmitteln pflanzlicher Herkunft verbreitet vorkommen. Glycoside sind chemische Verbindungen, bei denen ein Alkohol (R-OH) über eine glykosidische Bindung an einen Zuckerteil gebunden ist. Es handelt sich daher bei einem Glycosid um das Vollacetal eines Zuckers. Beispiele für typische Glycoside, die als sekundäre Pflanzenmetabolite vorkommen, sind die Glucosinolate. Grünkohl und andere Sorten der Pflanzenart *Brassica oleracea* besitzen einen verhältnismäßig hohen Gehalt an Glucosinolaten. Grünkohl enthält etwa 1 mg Glucosinolate pro Gramm Frischgewicht (Rouzaud G et al. Br J Nutr. 2003; 90(2):395-404). Über die Konzentration an Flavonoiden in Grünkohl sind recht unterschiedliche Meinungen in der Literatur zu finden. Untersuchungen des Erfinders zeigen, dass die Trockenmasse von Grünkohl 1,5% bis 2% Flavonoide enthalten kann. Über die genauen Mengen der jeweiligen Glucosinolate bzw. Flavonoide ist relativ wenig bekannt. Auch der Kulturapfel, und insbesondere seine Früchte (Äpfel) und die Rinde des Apfelbaumes, enthalten verhältnismäßig hohe Konzentrationen an Polyphenolen (inklusive Flavonoide). Je nach Sorte kann Apfelsaft über 400 mg Polyphenole pro Liter enthalten (Will, F., et al., 2006, LWT - Food Science and Technology, 40(8):1344-1351).

In einer Ausführungsart enthält die Zusammensetzung einen Extrakt aus Grünkohl, vorzugsweise Grünkohlblättern, und einen Extrakt aus den Äpfeln einer Pflanze der Gattung *Malus,* vorzugsweise *Malus domesticus.* In einer anderen Ausführungsart enthält die Zusammensetzung einen Extrakt aus Grünkohl, vorzugsweise Grünkohlblättern, der mit Phlorizin vermischt wurde.

Sofern die vorliegende Erfindung die Verwendung von pflanzlichen Extrakten betrifft, ist es bevorzugt, dass diese Extrakte die in den Pflanzenzellen enthaltenden Polyphenole, Flavonoide und/oder Glycoside enthalten. In einer bevorzugten Ausführungsform sind die Polyphenole, Flavonoide und/oder Glycoside in dem Extrakt oder in den Extrakten aufkonzentriert. Sofern für die Herstellung der erfindungsgemäßen Zusammensetzungen Phlorizin-haltige Extrakte verwendet werden, so können auch diese entsprechend behandelt werden, um das Phlorizin aufzukonzentrieren. Verfahren zur Aufkonzentrierung dieser Substanzen sind dem Fachmann hinreichend bekannt und werden z.B. in Will et al. (2006, LWT - Food Science and Technology, 40(8):1344-1351) beschrieben. Eine Aufkonzentrierung der Polyphenole, Flavonoide und/oder Glycoside oder von Phlorizin kann beispielsweise durch Verwendung von Säulenchromatographieverfahren erreicht werden. Zu diesem Zweck können z.B. verschiedene Adsorbersäulen eingesetzt werden. Die Chromatographiesäulen werden mit dem Ausgangsextrakt beladen, durch Spülen mit geeigneten Lösungen von unerwünschten Bestandteilen befreit und anschließend in aufkonzentrierter Form von der Säule eluiert. Zum Beispiel werden nach Beladen von Adsorbersäulen mit unkonzentriertem Extrakt durch Spülen mit demineralisiertem Wasser hydrophile Substanzen (Salze, Aminosäuren, Peptide, Zucker etc.) abgereichert. In dem mit 95% Ethanol eluiertem Extrakt sind anschließend Polyphenole angereichert.

Das erfindungsgemäße Verfahren erlaubt somit ausgehend von Grünkohl-Pflanzen und Phlorizin bzw. Phlorizin-enthaltenen Pflanzen die Herstellung von Zusammensetzungen mit einem hohen Anteil an gesundheitsfördernden Polyphenolen, Flavonoiden und/oder Glycosiden, die eine inhibierende Wirkung auf den Glucose-Transporter SGLT-1 ausüben. Die erfindungsgemäß erhältlichen Extrakte können sowohl in trockener als auch in flüssiger Form hergestellt werden.

In einem Aspekt betrifft die vorliegende Erfindung die Verwendung einer wie oben beschriebenen Zusammensetzung zur Herstellung einer pharmazeutischen Zusammensetzung, eines diätetischen Lebensmittels oder eines Nahrungsergänzungsmittels. In einem noch weiteren Aspekt betrifft die vorliegende Erfindung somit auch eine pharmazeutische Zusammensetzung, ein diätetisches Lebensmittel oder ein Nahrungsergänzungsmittel, die/das eine wie oben beschriebene Zusammensetzung umfasst, welche mindestens einen Extrakt aus einer Pflanze der Gattung *Brassica,* wobei es sich bei der Pflanze der Gattung *Brassica* um Grünkohl handelt, und mehr als 1% (w/w) Phlorizin umfasst. Die pharmazeutische Zusammensetzung, das diätetische Lebensmittel oder das Nahrungsergänzungsmittel eignen sich zur Verwendung in einem Verfahren zur Behandlung und/oder Prophylaxe einer hyperglykämischen Erkrankung, vorzugsweise ausgewählt aus der Gruppe bestehend aus Fettsucht, Diabetes oder eine von Diabetes verursachte Folgeerkrankung, und insbesondere zur Behandlung und/oder Prophylaxe der Diabetes vom Typ II.

Eine die erfindungsgemäße Zusammensetzung umfassende pharmazeutische Zusammensetzung kann für die orale, parenterale oder topische Darreichung formuliert sein. Eine solche pharmazeutische Zusammensetzung kann mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Die pharmazeutischen Zusammensetzungen können beispielsweise in Form von Granulaten, Pulvern, Tabletten, Kapseln, Sirup, Suppositorien, Injektionen, Emulsionen, Suspensionen oder Lösungen vorliegen. Die Zusammensetzungen können für verschiedene Verabreichungsarten formuliert sein, beispielsweise für die orale, parenterale, topische, bukkale, sublinguale, transmukosale, rektale, subkutane intrathekale, intravenöse, intramuskuläre, intraperitoneale, nasale, intraokulare oder intraventrikuläre Verabreichung. Die Formulierung als orale, parenterale oder topische Zusammensetzung ist besonders bevorzugt. In einer besonders bevorzugten Ausführungsform ist die Zusammensetzung für die orale Verabreichung formuliert. Die pharmazeutischen Zusammensetzungen können auch als Retardmittel formuliert werden.

Für die orale, bukkale und sublinguale Verabreichung werden in der Regel feste Formulierungen wie, z.B. Pulver, Suspensionen, Granulate, Tabletten, Pillen, Kapseln und Gelcaps verwendet. Diese können beispielsweise hergestellt werden, indem man die wirksamen Bestandteile (den Brassica-Extrakt und Phlorizin) mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropylmethylcellulose, Casein, Albumin, Mannit, Dextran, Saccharose, Lactose, Sorbitol, Stärke, Agar, Alginate, Pectine, Kollagen, Glyceride oder Gelatine verwendet werden. Ferner können Zusammensetzungen für die orale Verabreichung Antioxidantien (z.B. Ascorbinsäure, Tocopherol oder Cystein), Gleitmittel (z.B. Magnesiumstearat), Konservierungsmittel (z.B. Paraben oder Sorbinsäure), Geschmacksverbesserer, Sprengmittel, Bindemittel, Verdicker, Farbstoffe und ähnliche Substanzen umfassen.

Flüssige Formulierungen der erfindungsgemäßen Extrakte und Zusammensetzungen, die sich für die orale Verabreichung eignen, können beispielsweise als Emulsion, Sirup, Suspension oder Lösungen vorliegen. Diese Formulierungen können unter Verwendung einer sterilen Flüssigkeit als Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Für den Gebrauch in flüssigen Dosisformen geeignete Öle umfassen beispielsweise Olivenöl, Sesamöl, Erdnussöl, Rapsöl und Maisöl. Geeignete Alkohole umfassen Ethanol, Isopropylalkohol, Hexadecylalkohol, Glycerin und Propylenglykol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

Die vorliegende Erfindung betrifft ferner auch ein diätetisches Lebensmittel oder ein Nahrungsergänzungsmittel, das eine wie oben definierte Zusammensetzung umfasst, d.h. eine Zusammensetzung, die mindestens einen Extrakt aus einer Pflanze der Gattung *Brassica,* wobei es sich bei der Pflanze der Gattung *Brassica* um Grünkohl handelt, und mehr als 1% (w/w) Phlorizin enthält. Das diätetisches Lebensmittel oder Nahrungsergänzungsmittel ist insbesondere für die Behandlung und/oder Prophylaxe der wie oben definierten Fettsucht, Diabetes (insbesondere Diabetes Typ II) oder einer von Diabetes verursachten Folgeerkrankung geeignet. Es ist besonders bevorzugt, dass der mindestens eine Brassica-Extrakt aus den Blättern der jeweiligen Brassica-Pflanze gewonnen wurde. Ebenfalls vorzugsweise stammt das Phlorizin aus mindestens einem Phlorizin-enthaltenen, pflanzlichen Extrakt. Der mindestens eine Phlorizin-enthaltene Extrakt stammt vorzugsweise von einer Pflanze der Gattung *Malus,* insbesondere aus einer Pflanze der Spezies *Malus domesticus.* Es ist besonders bevorzugt, dass mindestens ein Extrakt aus einer Pflanze der Gattung *Malus* aus den Äpfeln oder der Rinde einer Malus-Pflanze gewonnen wurde. Gemäß einer weiteren bevorzugten Ausführungsform wurde der mindestens eine Extrakt aus einer Brassica-Pflanze aus den Blättern der Brassica-Pflanze gewonnen und das Phlorizin wurde dem Extrakt in isolierter und/oder gereinigter Form zugegeben.

### KURZE BESCHREIBUNG DER FIGUREN

Figur 1 zeigt die Wirkung einer Zusammensetzung, die einen Grünkohlextrakt und einen Apfelextrakt enthält, auf die Blutglucose. Gezeigt ist der Verlauf der Glucosemessungen bei Probanden, die Weißbrot bzw. Weißbrot mit einer Mischung aus Grünkohlextrakt (1,8 g) und Apfelextrakt (1,8 g) zu sich genommen haben.
Figur 2 zeigt die Wirkung des Apfelextrakts auf die Blutglucosekonzentration der Probanten.
Figur 3 zeigt Wirkung des Grünkohlextrakts auf die Blutglucosekonzentration der Probanten.

Die Erfindung wird nunmehr durch die nachfolgenden Beispiele veranschaulicht, wobei diese die Erfindung in keiner Weise beschränken.

### BEISPIELE

Die nachfolgenden Beispiele beschreiben die physiologische Wirkung der erfindungsgemäßen Zusammensetzungen:

### Beispiel 1

Es wurden verschiedene Extrakte aus Blättern von *Brassica ole*racea und Äpfeln (*Malus*) hergestellt.

### Ansatz (A): Brassica-Extrakt

6 Kilogramm Blätter von *Brassica oleracea* convar. *acephala* var. *sabellica* (Grünkohl) wurden mit einem Cutter zerkleinert, und der pH-Wert der resultierenden Maische wurde mit Ascorbinsäure auf pH 5 eingestellt. Der enzymatische Aufschluss erfolgte mit den Enzymen Cellulase (0,025 ml Vegazym HC, 11000 Units/ml, Fa. Erbslöh) und Pektinase (0,025 ml Vegazym P, 40 Units/ml, Fa. Erbslöh) bei 37°C. Die Inkubationszeit betrug 4 Stunden. Anschließend wurde der Ansatz zentrifugiert und die Polyphenole (inklusive der vorhandenen Flavonoide) aus dem Überstand, wie von Will et al. (2006, LWT - Food Science and Technology, 40(8):1344-1351) beschrieben, angereichert: Hierbei wird eine Adsorbersäule (XAD 16HD, Fa. Rohm & Haas, Frankfurt) mit dem Überstand beladen. Durch Spülen mit demineralisiertem Wasser werden hydrophile Substanzen (Salze, Aminosäuren, Peptide, Zucker etc.) abgereichert. Polyphenole dagegen werden durch Elution mit Ethanol (95%) angereichert. Die nachfolgende Gefriertrocknung der eluierten Fraktionen liefert ein Pulver (ca. 80 g), das für nachfolgende Studien verkapselt (Gelatine) wurde.

### Ansatz (B): Malus-Extrakt

6 Kilogramm Äpfel (*Malus domestica*) wurden mit einem Cutter zerkleinert, und der pH-Wert der resultierenden Maische wurde mit Ascorbinsäure auf pH 5 eingestellt. Der enzymatische Aufschluss erfolgte mit den Enzymen Cellulase (0,025 ml Vegazym HC, 11000 Units/ml, Fa. Erbslöh) und Pektinase (0,025 ml Vegazym P, 40 Units/ml, Fa. Erbslöh) bei 37°C. Die Inkubationszeit betrug 4 Stunden. Anschließend wurde der Ansatz zentrifugiert und die Polyphenole (inklusive Flavonoide) aus dem Überstand, wie in der Veröffentlichung von Will et al. (2006, LWT - Food Science and Technology, 40(8):1344-1351) beschrieben, angereichert: Hierbei wird eine Adsorbersäule (XAD 16HD, Fa. Rohm & Haas, Frankfurt) mit dem Überstand beladen. Durch Spülen mit demineralisiertem Wasser werden hydrophile Substanzen (Salze, Aminosäuren, Peptide, Zucker etc.) abgereichert. Polyphenole dagegen werden durch Elution mit Ethanol (95%) angereichert. Die nachfolgende Gefriertrocknung der eluierten Fraktionen liefert ein Pulver (ca. 25 g), das für nachfolgende Studien verkapselt (Gelatine) wurde. Der Extrakt enthielt etwa 15% Phlorizin, d.h. 1,8 g Apfelextrakt enthielten etwa 0,27 g Phlorizin.

### Ansatz (C): Extraktmischung

Die in (A) und (B) beschriebenen Extrakte wurden 1:1 gemischt und nach der Mischung für nachfolgende Studien verkapselt. Die biologischen Eigenschaften der Extrakte wurden im Rahmen von humanen Studien getestet:

### Studiendesign

Für die humanen Studien wurden jeweils 10 Probanden getestet. Jeder Proband erhielt an einem Testtag (Testtag 1) nur Weißbrot und an einem weiteren Testtag einen der beiden Extrakte bzw. die Extraktmischung zusammen mit Weißbrot. Es handelte sich um eine "Crossover-Studie".
Testtag 1: Jeder Proband erhielt 104 g Weißbrot (ohne Rinde), die innerhalb von 10 Minuten zusammen mit 150 ml Wasser verzehrt wurden (Zeitpunkt 0). Den Probanden wurde 10 Minuten vor der Einnahme des Weißbrotes Blut über eine Kanüle abgenommen, und die Blutglucosewerte wurden mit Hilfe des HemoCue® Glukose System 2.0 der Fa. HemoCue GmbH, 63762 Großostheim, bestimmt. Zum Zeitpunkt 0 und in Abständen von jeweils 15 Minuten erfolgten vier weitere Glucosebestimmungen. Anschließend wurden im Abstand von 30 Minuten die Blutglucosewerte bestimmt. Die letzte Messung erfolgte 180 Minuten nach Einnahme des Weißbrotes.
Testtag 2: Durchführung wie am Testtag 1, allerdings wurde der Extrakt bzw. die Extraktmischung 10 Minuten vor Einnahme des Weißbrotes eingenommen.

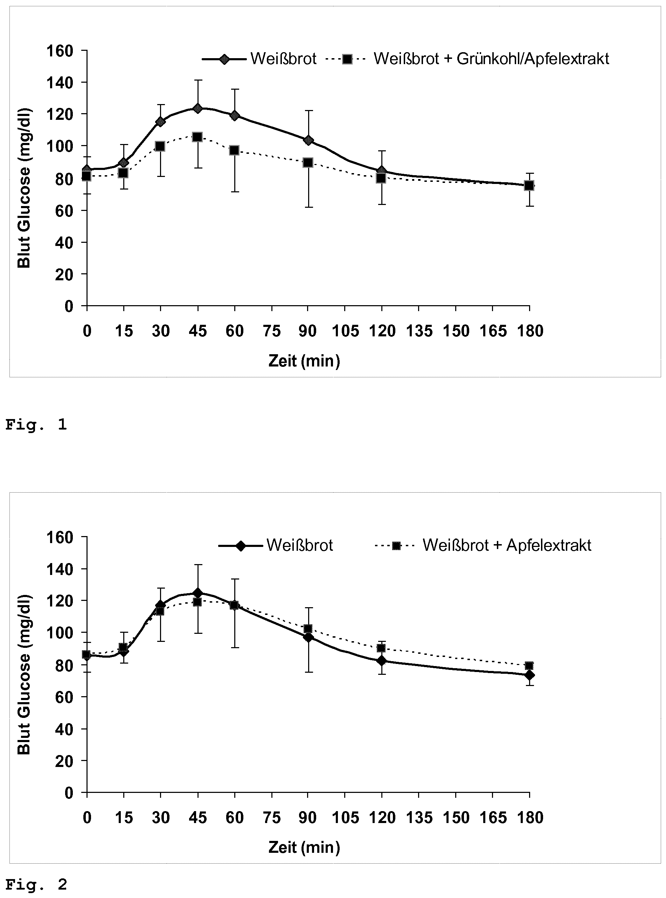

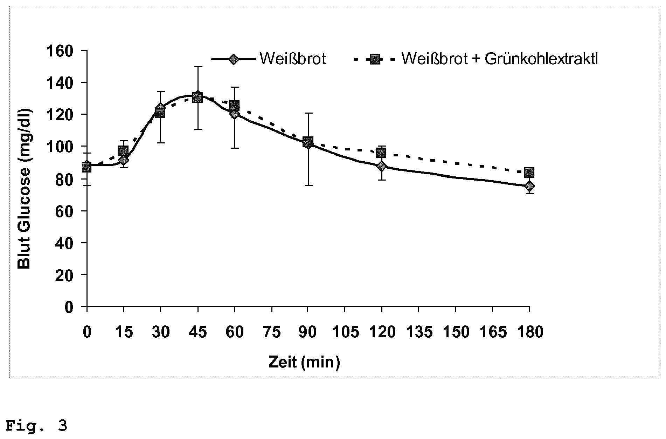

Der modulierende Einfluss auf die Blutglucose der getrennt verabreichten *Malus-* bzw. Brassica-Extrakte und der Extraktmischung ist in den Abbildungen 1-3 dargestellt. Abb. 1 zeigt den Verlauf der Glucosemessungen bei den Probanden, die Weißbrot bzw. Weißbrot mit einer Mischung aus Grünkohlextrakt (1,8 g) und Apfelextrakt (1,8 g) zu sich genommen haben. Die Einnahme der Extraktmischung hatte eine signifikante Wirkung, da der Glucoseanstieg nach Einnahme gegenüber der Kontrolle (nur Weißbrot) deutlich geringer war (p = 0,02). Abb. 2 zeigt die Wirkung des getrennt verabreichten Apfelextrakts (3,6 g); Abb. 3 zeigt die Wirkung des getrennt verabreichten Grünkohlextrakts (3,6 g). In beiden Fällen gab es lediglich eine Tendenz, aber keine signifikante Wirkung auf die Blutglucosewerte (p> 0,05). Diese Daten zeigen, dass die Extraktmischung eine signifikante, inhibierende Wirkung auf die Aufnahme von Glucose im Darm aufweist, während die getrennt verabreichten Extrakte bei in entsprechender Menge eine deutlich schwächere Wirkung aufweisen. Offenbar liegt hier ein synergistischer Effekt der beiden Extrakte vor.

## Patentansprüche

1. Zusammensetzung, umfassend:
(a) mindestens einen Extrakt aus einer Pflanze der Gattung *Brassica,* und
(b) mehr als 1% (w/w) Phlorizin,
wobei es sich bei der Pflanze der Gattung *Brassica* um Grünkohl handelt.

2. Zusammensetzung nach Anspruch 1, wobei der Extrakt der Pflanze der Gattung *Brassica* aus den Blättern der *Brassica* Pflanze gewonnen wurde.

3. Zusammensetzung nach einem der Ansprüche 1-2, wobei das Phlorizin in Form eines Phlorizin-haltigen pflanzlichen Extrakts vorliegt.

4. Zusammensetzung nach Anspruch 3, wobei der Phlorizin-haltige pflanzliche Extrakt aus mindestens einer Pflanze der Familie *Rosaceae* gewonnen wurde, und wobei die Pflanze vorzugsweise einer Gattung angehört, die ausgewählt ist aus der Gruppe bestehend aus *Malus, Pyrus* oder *Prunus.*

5. Zusammensetzung nach Anspruch 4, wobei der Phlorizin-haltige pflanzliche Extrakt aus mindestens einer Pflanze der Familie *Verbenaceae* gewonnen wurde, wobei die Pflanze vorzugsweise der Gattung *Lippia* angehört.

6. Zusammensetzung nach einem der Ansprüche 3-5, wobei der Phlorizin-haltige pflanzliche Extrakt aus der Rinde, den Früchten oder den Blättern der Pflanze gewonnen wurde.

7. Zusammensetzung nach einem der Ansprüche 1-6, wobei der Extrakt der Pflanze der Gattung *Brassica* und/oder der Extrakt der Pflanze aus der Familie der *Rosaceae* oder *Verbenaceae* Flavonoide, Glycoside und/oder Polyphenole enthält.

8. Zusammensetzung nach einem der Ansprüche 1-7, zur Verwendung in einem Verfahren zur Behandlung und/oder Prophylaxe einer hyperglykämischen Erkrankung, vorzugsweise ausgewählt aus der Gruppe bestehend aus Fettsucht, Diabetes oder eine von Diabetes verursachte Folgeerkrankung.

9. Verwendung einer Zusammensetzung nach einem der Ansprüche 1-7 zur Herstellung einer pharmazeutischen Zusammensetzung, eines diätetischen Lebensmittels oder eines Nahrungsergänzungsmittels.

10. Pharmazeutische Zusammensetzung, diätetisches Lebensmittel oder Nahrungsergänzungsmittel, die/das eine Zusammensetzung nach einem der Ansprüche 1-7 umfasst.

11. Pharmazeutische Zusammensetzung, diätetisches Lebensmittel oder Nahrungsergänzungsmittel nach Anspruch 10, zur Verwendung in einem Verfahren zur Behandlung und/oder Prophylaxe einer hyperglykämischen Erkrankung, vorzugsweise ausgewählt aus der Gruppe bestehend aus Fettsucht, Diabetes oder eine von Diabetes verursachte Folgeerkrankung.

12. Pharmazeutische Zusammensetzung, diätetisches Lebensmittel oder Nahrungsergänzungsmittel zur Verwendung nach Anspruch 11, **dadurch gekennzeichnet, dass** der Diabetes vom Typ II ist.

13. Pharmazeutische Zusammensetzung zur Verwendung nach einem der Ansprüche 11-12, **dadurch gekennzeichnet, dass** die Zusammensetzung für die orale, parenterale oder topische Darreichung formuliert ist.

## Claims

1. Composition comprising:
(a) at least one extract of a plant of the genus *Brassica,* and
(b) more than 1% (Gew./Gew.) phlorizin,
wherein the plant of the genus *Brassica* is kale.

2. A composition according to claim 1, wherein the extract of the plant of the genus *Brassica* was obtained from the leaves of the *Brassica* plant.

3. A composition according to any of claims 1-2, wherein the phlorizin is present in the form of a phlorizin-containing plant extract.

4. Composition according to claim 3, wherein the phlorizin-containing plant extract was obtained from at least one plant of the family *Rosaceae,* and wherein the plant preferably belongs to a genus selected from the group consisting of *Malus, Pyrus* or *Prunus.*

5. Composition according to claim 4, wherein the phlorizin-containing plant extract was obtained from at least one plant of the family *Verbenaceae,* wherein the plant preferably belongs to the genus *Lippia.*

6. A composition according to any of claims 3-5, wherein the phlorizin-containing plant extract was obtained from the bark, the fruits or the leaves of the plant.

7. A composition according to any of claims 1-6, wherein the extract of the plant of the genus *Brassica* and/or the extract of the plant of the family *Rosaceae* or *Verbenaceae* contains flavonoids, glycosides and/or polyphenols.

8. Composition according to any of claims 1-7 for use in a method of treatment and/or prophylaxis of a hyperglycemic disease, preferably selected from the group consisting of obesity, diabetes or a diabetes-related secondary disease.

9. Use of a composition according to any of claims 1-7 for the preparation of a pharmaceutical composition, a dietetic food or a nutritional supplement.

10. Pharmaceutical composition, dietetic food or nutritional supplement comprising a composition according to any of claims 1-7.

11. Pharmaceutical composition, dietetic food or nutritional supplement according to claim 10 for use in a method of treatment and/or prophylaxis of a hyperglycemic disease, preferably selected from the group consisting of obesity, diabetes or diabetes-related secondary disease.

12. Pharmaceutical composition, dietetic food or nutritional supplement for use according to claim 11, wherein the diabetes is diabetes Type II.

13. Pharmaceutical composition for use according to any one of claims 11-12, wherein the composition is formulated for oral, parenteral or topical administration.

## Revendications

1. Composition, comprenant:
(a) au moins un extrait d'une plante appartenant au genre *Brassica,* et
(b) plus de 1 % (p/p) en poids de phlorizine,
dans laquelle pour ce qui est de la plante appartenant au genre *Brassica* il s'agit du chou frisé.

2. Composition selon la revendication 1, dans laquelle l'extrait de la plante appartenant au genre *Brassica* a été obtenu à partir des feuilles de la plante *Brassica.*

3. Composition selon l'une quelconque des revendications 1 et 2, dans laquelle la phlorizine est présente sous forme d'un extrait végétal contenant de la phlorizine.

4. Composition selon la revendication 3, dans laquelle l'extrait végétal contenant de la phlorizine a été obtenu à partir d'au moins une plante de la famille des *Rosaceae*, et dans laquelle la plante appartient de préférence à un genre qui est choisi dans l'ensemble constitué par les genres *Malus, Pyrus* et *Prunus.*

5. Composition selon la revendication 4, dans laquelle l'extrait végétal contenant de la phlorizine a été obtenu à partir d'au moins une plante de la famille des *Verbenaceae,* la plante appartenant de préférence au genre *Lippia.*

6. Composition selon l'une quelconque des revendications 3 à 5, dans laquelle l'extrait végétal contenant de la phlorizine a été obtenu à partir des écorces, des fruits ou des feuilles de la plante.

7. Composition selon l'une quelconque des revendications 1 à 6, dans laquelle l'extrait de la plante appartenant au genre *Brassica* et/ou l'extrait de la plante de la famille des *Rosaceae* ou *Verbenaceae* contient des flavonoïdes, des glycosides et/ou des polyphénols.

8. Composition selon l'une quelconque des revendications 1 à 7, destinée à l'utilisation dans un procédé pour le traitement et/ou la prophylaxie d'une maladie hyperglycémique, choisie de préférence dans l'ensemble constitué par l'adiposité, le diabète ou une maladie secondaire causée par le diabète.

9. Utilisation d'une composition selon l'une quelconque des revendications 1 à 7, pour la fabrication d'une composition pharmaceutique, d'un produit alimentaire diététique ou d'un complément alimentaire.

10. Composition pharmaceutique, produit alimentaire diététique ou complément alimentaire, qui comprend une composition selon l'une quelconque des revendications 1 à 7.

11. Composition pharmaceutique, produit alimentaire diététique ou complément alimentaire selon la revendication 10, destiné(e) à l'utilisation dans un procédé pour le traitement et/ou la prophylaxie d'une maladie hyperglycémique, choisie de préférence dans l'ensemble constitué par l'adiposité, le diabète ou une maladie secondaire causée par le diabète.

12. Composition pharmaceutique, produit alimentaire diététique ou complément alimentaire destiné(e) à l'utilisation selon la revendication 11, caractérisé(e) en ce que le diabète est du type II.

13. Composition pharmaceutique destinée à l'utilisation selon l'une quelconque des revendications 11 et 12, **caractérisée en ce que** la composition est formulée pour l'administration orale, parentérale ou topique.
